# EUROPEAN PATENT SPECIFICATION

(11) **EP 2 914 623 B1**
(45) Date of publication and mention of the grant of the patent: **28.12.2016**
(21) Application number: 13783920.5
(22) Date of filing: 29.10.2013
(51) Int. Cl.: C07K 14/735, A61K 38/17

(54) **A PHARMACEUTICAL COMPOSITION FOR USE IN TREATING OR PREVENTING EITHER ONE OR BOTH OF AN INFLAMMATORY DISEASE AND AN AUTOIMMUNE DISEASE**
PHARMAZEUTISCHE ZUBEREITUNG ZUR VERWENDUNG IN BEHANDLUNG ODER VORBEUGUNG VON ENTWEDER EINEM ODER MEHREREN INFLAMATORISCHEN ODER AUTOIMMUNEN KRANKHEITEN
COMPOSITION PHARMACEUTIQUE UTILISABLE DANS LE TRAITEMENT OU PRÉVENTION D'UNE OU PLUSIEURS MALADIES INFLAMMATOIRES OU AUTOIMMUNES

(30) Priority: 30.10.2012 US 201213663527; 31.10.2012 KR 20120122658
(43) Date of publication of application: 09.09.2015
(73) Proprietor: SuppreMol GmbH, 82152 Martinsried (DE)
(72) Inventor: BUCKEL, Peter, 82347 Bernried (DE); SONDERMANN, Peter, 82131 Stockdorf (DE); TILLMANNS, Sascha, 81243 München (DE); BERGER, Ingrid, 80636 München (DE)
(74) Representative: Schiweck, Weinzierl & Koch
(86) International application number: PCT/EP2013/072631
(87) International publication number: WO 2014/067959

(56) References cited:
- WO-A1-00/32767
- WO-A2-03/043648
- S E MAGNUSSON ET AL.: "Amelioration of collagen-induced arthritis by human recombinant soluble Fc-gamma-RIIb", CLINICAL IMMUNOLOGY, vol. 127, no. 2, 2008, pages 225-233, XP22595347, ACADEMIC PRESS, ISSN: 1521-6616

## Description

The present invention relates to a pharmaceutical composition for use in treating or preventing either one or both of an inflammatory disease and an autoimmune disease in a patient. Said pharmaceutical composition is for administering a therapeutically effective amount of a soluble Fcγ receptor (FcγR). Specific schedules and time points of administration allow a surprisingly effective treatment or prevention of a disease in the patient.

Specifically, the invention relates to a pharmaceutical composition for use in treating or preventing either one or both of an inflammatory disease and an autoimmune disease, wherein the pharmaceutical composition comprises soluble Fcγ receptor (FcγR) as active ingredient; and wherein the pharmaceutical composition is designed for administering a first dose of a therapeutically effective amount of soluble FcγR, followed by a safety period of several weeks, followed by a subsequent treatment cycle of at least two further administrations, wherein said therapeutically effective amount is effective to treat or prevent said disease in said patient.

WO 00/32767 describes soluble Fc receptors (FcRs) which are composed of only the extracellular part of the receptor and are not glycosylated. Due to the absence of the transmembrane domain and of the signal peptide, these proteins are present in a soluble form and not bound to cells. Furthermore the FcRs described in WO 00/32767 can be produced recombinantly and have been suggested for the treatment of autoimmune diseases due to their ability to bind the Fc part of antibodies without interfering with other components of the immune system. WO 00/32767 additionally describes the crystal structure of certain FcRs and the possibility of finding substances that inhibit the interaction of IgG with FcRs with the aid of these crystal structures. The elucidation of the crystal structure enables finding such inhibitors by screening the databases using available computer programs.

The invention which was defined in WO 03/043648 further developed the findings of WO 00/32767 and provides treatment methods especially for diseases like multiple sclerosis (MS), systemic lupus erythematosus (SLE), and rheumatoid arthritis (RA) and also for diseases with an elevated level of natural killer cells. When said receptors were produced recombinantly in prokaryotes and therefore were unglycosylated the inventors of WO 03/043648 surprisingly found that although the unglycosylated proteins were expected to be poorly soluble, the receptors could be purified with high concentrations of FcγR in a soluble form.

When said receptors were produced recombinantly in prokaryotes and therefore were unglycosylated the inventors of WO 03/043648 surprisingly found that although the unglycosylated proteins were expected to be poorly soluble, the receptors could be purified with high concentrations of FcγR in a soluble form.

WO 03/043648 and other publication documents describe that FcRs play an important role in defense reactions of the immune system. When pathogens have entered the blood circulation they are bound by immunoglobulins, also known as antibodies. Since the immune response to a pathogen is poylclonal, a multitude of antibodies bind to a pathogen, leading to the formation of a so called immune-complex (IC). ICs are subsequently phagocytised by specialized effector cells (e.g. phagocytes or macrophages) of the immune system and thus removed from the circulation. The phagocytosis is mediated by the binding of the Fc-part of the antibodies, forming the IC together with the pathogen, to FcRs on the aforementioned effector cells. Other effector cells of the immune system, such as natural killer cells, eosinophils and mast cells also carry FcRs on their surface which upon binding of immune complexes release stored mediators such as growth factors or toxins that support the immune response.
The FcRs of these effector cells also function as signal-transducing molecules that specifically bind immunoglobulins of various isotypes during the humoral immune response. In addition FcRs expressed on natural killer cells play a fundamental role in the destruction of antibody-coated target cells ("antibody-dependent cell-mediated cytotoxicity", ADCC).

However, in addition to the positive effects of FcRs in the defense against pathogens, overshooting reactions caused by the presence of auto-antibodies in patients may also occur which result in an undesired stimulation of the immune system which manifests itself especially as inflammatory or autoimmune diseases. Such immune reactions directed against the body's own substances remain a major medical problem and although there are approaches for treating them, these approaches are not equally effective in every patient.

All members of the FcγR-family are integral membrane glycoproteins, possessing extracellular domains related to a C2-set of immunoglobulin-related domains, having a single membrane spanning domain and an intracytoplasmic domain of variable length. There are three known Fcγ receptor forms, designated FcγRI (CD64), FcγRII (CD32), and FcγRIII (CD16). This invention specifically focuses on FcγRII (CD32).

FcγRII proteins are 40KDa integral membrane glycoproteins which bind only bind the complexed IgG. These receptors are the most widely expressed FcγRs, present on all hematopoietic cells, including monocytes, macrophages, B cells, NK cells, neutrophils, mast cells, and platelets. There are three human FcγRII genes (FcγRII-a, FcγRII-b, FcγRII-c), all of which bind IgG in aggregates or immune complexes.
Inflammation is a process by which the body's white blood cells react to infection by foreign substances, such as bacteria and viruses. It is usually characterized by pain, swelling, warmth and redness of the affected tissue. Effector substances known as cytokines and prostaglandins control this process, and are released in an ordered and self-limiting cascade into the blood or affected tissues. The release of such effector substances increases the blood flow to the area of injury or infection. Some of the effector substances cause a leak of fluid into the tissues, resulting in swelling. This protective process may stimulate nerves and cause pain. These changes, when occurring for a limited period in the relevant area, work to the benefit of the body.

In autoimmune diseases the patient's immune system has lost the ability to discriminate between body-own ("self") and foreign proteins. In consequence, antibodies are generated that recognize "self"-proteins and form immune complexes which continuously activate the immune system because the "self"-protein is permanently produced and recognized as foreign. This chronic condition can persist for years leading in the end to severe organ damage and possibly to the death of the patient. There are many different autoimmune disorders which affect the body in various ways. For example, the brain is affected in individuals with multiple sclerosis, the gut is affected in individuals having Crohn's disease, and the synovium, bone and cartilage of various joints are affected in individuals suffering from rheumatoid arthritis. As autoimmune disorders progress destruction of one or more types of body tissues, abnormal growth of an organ, or changes in organ function may result. The autoimmune disorder may affect a single organ or tissue type or may affect multiple organs and tissues. Organs and tissues commonly affected by autoimmune disorders include red blood cells, blood vessels, connective tissues, endocrine glands (e. g., the thyroid or pancreas), muscles, joints, and the skin.

Examples of inflammatory and/or autoimmune disorders include, but are not limited to, primary immune thrombocytopenia (ITP), systemic lupus erythematosus (SLE), rheumatoid arthritis (RA), autoimmune hemolytic anaemia (AIHA), diabetes, Pemphigus vulgaris, Hashimoto's thyroiditis, autoimmune inner ear disease myasthenia gravis, pernicious anemia, Addison's disease, dermatomyositis, Sjogren's syndrome, dermatomyositis, multiple sclerosis, Reiter's syndrome, Graves disease, autoimmune hepatitis, familial adenomatous polyposis and ulcerative colitis.
Primary Immune Thrombocytopenia (ITP) is an autoimmune disorder characterized by a low platelet count (thrombocytopenia) of unknown aetiology. The immune system of ITP patients produces antibodies directed against their own platelets. These auto-antibodies form immune-complexes with platelets, which are subsequently recognized by Fc-gamma receptors (FcγRs) expressed on immune cells. This interaction triggers a wide range of responses which includes up-take, processing, antigen presentation as well as initiation of cellular cytotoxicity and release of inflammatory and immune mediators. As a result the platelet count in the blood is decreased and patients start suffering from bruising and potentially fatal spontaneous bleedings (purpura). The reason why the body reacts against its own platelets is currently unknown.

Primary Immune Thrombocytopenia is defined by platelet counts < 100,000/µLⁱ and is characterized clinically by an increased tendency to bruise. Clinically, ITP often presents as spontaneous bleeding in individuals with platelet counts of less than 20,000/µL. Subjects with platelet counts < 10,000/µL may present with severe cutaneous bleeding, gingival bleeding, epistaxis, hematuria or menorrhagia. Some bleeding risk is present in subjects with platelet counts between 30,000/µL and 50,000/µL depending on the coexisting factors for bleedingⁱⁱ

Primary Immune Thrombocytopenia affects between 1 and 4 in 10,000 persons in the European Union, which corresponds to 50,000-200,000 persons in totalⁱⁱⁱ. Children account for about half of all cases. In the United States, the ITP prevalence is estimated between 1 and 2.4 per 10,000 corresponding to up to 72,300 patients in total^{iv},^{v}. Children developing ITP experience acute disease followed by complete spontaneous remission, in most cases. In contrast, adults will often develop chronic ITP, a disease characterized by persistent moderate to severe thrombocytopenia that puts them at risk for bleeding with trauma and can also result in spontaneous hemorrhage of variable severity.

Diagnosis of ITP in adults is based on confirmed thrombocytopenia together with exclusion of other causes for the thrombocytopenia based on physical examination, complete blood count and blood smear results^{vi}. Assays for antiplatelet antibodies are not sufficiently sensitive or specific to justify their routine use in the diagnosis of ITP^{vii}.

The American Society of Hematology and the British Society for Hematology recommend initial treatment consisting of a full course of steroids or intravenous immunoglobulin. The initial treatment in adults with severe ITP is prednisone, usually at a dose of 1 mg/kg/day for 2 to 4 weeks. Patients who do not adequately respond to first line treatment are candidates for splenectomy. Two thirds of subjects with ITP who undergo splenectomy will achieve a normal platelet count^{viii}. Although splenectomy is a routine surgical procedure, it is endowed with a 0.8% risk of surgical mortality and a complication rate of 12%^{ix}. Overall, the use of splenectomy for ITP is declining.

Subjects who do not respond to the first and second line treatment are classified as having chronic refractory ITP and this happens for 11-35% of ITP subjects. The treatment options available are:
High dose steroids: Oral dexamethasone of short duration in cycles and parenteral methylprednisolone. The response to the latter is faster therefore this is indicated when the platelet count needs to be increased as a priority. This response is transitory and requires maintenance therapy in the form of oral steroids.
High doses of IVIG (intravenous immunoglobulin): A small number of studies have shown that IVIG is effective and raises platelet count faster than steroids in adults with ITP. The doses used varied but 1 mg/kg/day for 2 days was generally recommended^{x}. The response is usually transitory but the infusions of IVIG can be repeated.
Intravenous anti-D: In one study this therapy has been shown to elevate the platelet counts in 79-90% of adults and lasted up to 3 weeks in 50% of those who responded^{xi}. This treatment is only effective in Rh D-positive non-splenectomised patients.
Vinca alkaloids: Vincristine or vinblastine, given i.v., can cause an increase in platelet count lasting 1-3 weeks. 50% of splenectomised patients respond. This response is sustained in only a small proportion of subjects^{xii}.
Danazol: When administered at 200 mg 2-4 times daily for more than 2 months it resulted in a 60% response rate. It can be continued for over a year with less toxicity than long-term steroids. Age, sex, and the status of the spleen influence the responses^{xiii}.
Immunosuppressive agents: Azathioprine and cyclophosphamide have been successfully used (up to 25% of patients showed sustained response) but are slow acting and need long-term administration. Cyclosporin A can be given alone or with prednisone but carries a substantial risk of serious adverse reactions. Dapsone: Administered orally for several weeks at a dose of 75 to 100 mg, resulted in a remission in half of the treated subjects but it was less effective in subjects with severe ITP and in splenectomised subjects^{xiv}.

Experimental agents which have been used with good effect include thrombopoietic agents as well as rituximab or mycophenolate mofetil.

Rituximab, romiplostim, and eltrombopag are potential agents that have demonstrated the ability to increase platelet counts in patients with chronic ITP. Quite recently, the thrombopoietin-receptor agonists romiplostim and eltrombopag have been approved by the FDA for the treatment of chronic ITP in splenectomised patients. Romiplostim is also approved in the EU.

Romiplostim, a subcutaneous injectable analog of thrombopoietin, and eltrombopag, an oral non-peptide molecule, are indicated in the US for the treatment of thrombocytopenia in patients with chronic ITP who have an insufficient response to corticosteroids, immunoglobulins, or splenectomy.

Response was seen in splenectomised and non-splenectomised patients, including those who had no sustained benefit from multiple other agents. Further investigations need to be performed to define the risks of long-term use of thrombopoietic stimulating agents and the benefit of these novel agents in comparison to other therapies that provide a durable response off therapy^{xv}.

Mycophenolate mofetil is a new immunosuppressive agent used in ITP. One study has shown 39% of patients achieved a sustained response. This agent may be a useful component of a combination therapy in patients with refractory ITP^{xvi}.

In subjects requiring emergency treatment for low platelet count associated with active bleeding, high doses of i.v. corticosteroids or IVIG are indicated and transfusion of random donor platelets may be appropriate.

In spite of these different approaches for treating an autoimmune disease, they still represent a major health impairment and therefore further treatment approaches are needed.

Accordingly, the object of the present invention was to provide a pharmaceutical composition for use in treating or preventing diseases like systemic lupus erythematosus (SLE), rheumatoid arthritis (RA), autoimmune hemolytic anaemia (AIHA) and preferably primary immune thrombocytopenia (ITP) as well as a corresponding method of treatment or prevention.

The object was solved by a pharmaceutical composition for use in treating and preventing either one or both of an inflammatory disease and an autoimmune disease, wherein the pharmaceutical composition comprises soluble Fcγ receptor (FcγR) as active ingredient; and wherein the pharmaceutical composition is designed for administering a first dose of a therapeutically effective amount of soluble FcγR, followed by a safety period of several weeks, and followed by a subsequent treatment cycle of at least two further administrations.

According to the present invention, the response to the therapy using the pharmaceutical composition and in accordance with the mentioned treatment schedule surprisingly is characterized by an increase of the median platelet count in ITP patients which persists during the 3 months follow-up period at the end of the therapy. In case the platelet count does not increase sufficiently the administration may be repeated.

As used herein, the terms "disorder" and "disease" are used interchangeably to refer to a condition in a subject. In particular, the term "autoimmune disease" is used interchangeably with the term "autoimmune disorder" to refer to a condition in a subject characterized by cellular, tissue and/or organ injury caused by an immunologic reaction of the subject to its own cells, tissues and/or organs. The term "inflammatory disease" is used interchangeably with the term "inflammatory disorder" to refer to a condition in a subject characterized by inflammation, preferably chronic inflammation. Autoimmune disorders may or may not be associated with inflammation. Moreover, inflammation may or may not be caused by an autoimmune disorder. Thus, certain disorders may be characterized as both autoimmune and inflammatory disorders.

In a preferred embodiment the inflammatory disease which can be treated with the pharmaceutical composition is Primary Immune Thrombocytopenia (ITP), Systemic Lupus Erythematosus (SLE), Rheumatoid Arthritis (RA), or Autoimmune Hemolytic Anaemia (AIHA).

As used herein, the term "treating" and analogous terms refer to a management and care of a patient and/or the combating of disease or disorder.

As used herein, the terms "prevent", "preventing" and "prevention" refer to the prevention of the recurrence or the onset of one or more symptoms of a disorder or disease, especially in individuals which have been analyzed to be susceptible or likely to develop the disease. Such analysis can for example take into account certain marker substances in body fluids of individuals or a known or suspected hereditary disposition or risk for developing such diseases.

As used herein, a "therapeutically effective amount" refers to an amount of the therapeutic active component or agent which is sufficient to treat or ameliorate a disease or disorder, to delay the onset of a disease or provides any therapeutical benefit in the treatment or management of a disease.

The therapeutically effective amount per administration provided herein is from 0.3 to 5000 mg/kg, preferably from 0.3 to 1000 mg/kg, preferably from 1 to 250 mg/kg, preferably from 1 to 100 mg/kg, preferably at least 3 mg/kg, preferably from 3 to 50 mg/kg, preferably from 5 to 36 mg/kg, preferably from 10 to 36 mg/kg, preferably from 8 to 25 mg/kg, more preferably from 12 to 20 mg/kg, in particular 12 mg/kg.

Thus, it is preferred that a person of about 100kg receives of about 1200mg and a person of about 75kg receives of about 900mg of soluble FcγR, respectively.

As used herein, a "safety period" refers to a period of time during which no further administration of the active ingredient of the pharmaceutical composition, i.e. the soluble Fc receptor, takes place. The safety period can be considered as a period of time which follows the first administration which effects a "priming" or activation of cell populations which respond to the administration of the active ingredient. Within such safety period, the primed or activated cell populations, especially B-cell- and/or T cell-populations, can expand or stabilize. Changes within such cell populations but also changes in the cytokine levels or other changes in hematological parameters known to the person skilled in the art, e.g. activation of thrombocytes etc. can be observed or determined by applicable tests for immune monitoring, like e.g. FACS analysis for changes in the B- or T-cell population. According to such immune monitoring with regard to the patient's response to the first administration of the active ingredient, the applicable dose of the pharmaceutical composition as well as the time lag between administrations in correspondence with the teaching of the present patent can be fixed or adjusted.

As used herein, the term "soluble Fcγ receptor" and analogous terms refer to the extracellular part of the Fcγ receptor which can be dissolved in a liquid.

The pharmaceutical composition used in present invention contains at least one soluble Fcγ receptor in an amount, which is suitable to treat and/or prevent said disease in a patient. In one embodiment, the soluble FcγR is derived from FcγRIIB type receptor. In another embodiment, the soluble FcγR is SM101. SM101 is a recombinant, soluble, non-glycosylated version of the FcγRIIB as described WO 00/32767 and WO 03/043648, respectively.

The Fcγ receptor of the present invention comprises at least one of the amino acid sequences as shown in SEQ ID NO:1 (SM101) or SEQ ID NO:3 (FcγRIIB). In one preferred embodiment no mutations are introduced into the constructs when extending the N-termini and/or C-termini of the stated sequences in order to prevent antigenicity. However, it is theoretically possible to also introduce mutations or deletions into the extended sequences provided that they do not result in an undesired antigenicity.

Moreover, the soluble FcγR is of human origin. It is further characterized by the absence of transmembrane domains and signal peptide, which is described in e.g. WO 00/32767 (A1).

As used herein, the term "soluble FcγR is of human origin" and analogous terms refer to the extracellular part of the Fcγ receptor which can be dissolved in a liquid and which is derived from human and not from other species.

The soluble FcγR of the present invention in encoded by at least one of a nucleic acid sequence according to SEQ ID NO:2 (SM101) or SEQ ID NO:4 (FcγRIIB). These sequences can be cloned into an expression vector to produce the corresponding soluble FcγR by means of recombinant expression.

As used herein, the terms "nucleic acids" and "nucleotide sequences" include DNA molecules (e.g., cDNA or genomic DNA), RNA molecules (e.g., mRNA), combinations of DNA and RNA molecules or hybrid DNA/RNA molecules, and analogs of DNA or RNA molecules. Such analogs can be generated using, for example, nucleotide analogs, which include, but are not limited to, inosine or tritylated bases. Such analogs can also comprise DNA or RNA molecules comprising modified backbones that lend beneficial attributes to the molecules such as, for example, nuclease resistance or an increased ability to cross cellular membranes. The nucleic acids or nucleotide sequences can be single-stranded, double-stranded, may contain both single-stranded and double-stranded portions, and may contain triple-stranded portions, but preferably is double-stranded DNA.

In one embodiment the FcγRs can be expressed in prokaryotes and subsequently purified and refolded according to the description of WO 00/32767. The receptors have the advantage that they can be highly concentrated and hence only small volumes are necessary to administer adequately effective amounts. Furthermore, the costs of the pharmaceutical compositions according to the invention are very low since their production by recombinant expression in for example prokaryotes is simple and results in highly-purified and highly-concentrated protein preparations. Said soluble FcγR is therefore recombinant, soluble and non-glycosylated FcγR.

In another embodiment FcγRs can be easily and unexpensively produced in high purity in eukaryotic expression systems. Useful systems include eukaryotes with a specialized apparatus for the production of extracellular proteins, e.g. B cells. Other possible eukaryotic expression systems include, but are not limited to, CHO or HEK cells. Said soluble FcγR is therefore recombinant, soluble and glycosylated FcγR.

According to the present invention the pharmaceutical composition is administered at least once, in particular once on day 1, followed by a safety period of several weeks.
The term "several" refers to at least 2 weeks, preferably 3 weeks, more preferably 4 weeks, most preferably 5 weeks. The term "several weeks" refers to at least 14 days, preferably 21 days, more preferably 28 days, most preferably 35 days.

The safety period is at least 2 weeks, preferably 2 to 8 weeks, more preferably 4 to 6 weeks and most preferably 5 weeks.

The term "weekly" refers to a time period of about 4 to about 10 days, preferably 5 to 9 days, more preferably 6 to 8 days, most preferably 7 days.

According to the present invention, the safety period is followed by further administrations of the soluble FcγR. In one preferred embodiment of the invention, additional administrations after the safety period are performed daily, wherein it is also preferred to perform 2 to 8, especially 4 to 6 further administrations on consecutive days. In an especially preferred embodiment, the administration of a first dose of the receptor is followed by a safety period of 3 to 5 weeks and followed up by five further administrations on consecutive days.

In another preferred embodiment of the present invention, the subsequent treatment cycle after the safety period comprises 2 to 7 weekly administrations, preferably between days 30 and 60, more preferably the subsequent treatment cycle after the safety period consists of 3 to 5 weekly administrations between days 35 and 60 and most preferably the subsequent treatment cycle are 4 weekly administrations between days 35 and 56.

According to the present invention, 1 to 250 mg/kg active ingredient are administered as a first dose but also as subsequent treatment doses (amount for dose administered) to the patient (mg active ingredient per kg body weight).

In a preferred embodiment 5 to 40 mg/kg are administered, on day 1, followed by a 4 to 6 week safety period, followed by a subsequent treatment cycle of 3 to 5, weekly administrations of a further 5 to 40 mg/kg between days 35 and 60.

In a preferred embodiment 12 to 20mg/kg are administered on day 1, followed by a 5 week safety period, followed by a subsequent treatment cycle of 4 weekly administrations between days 35 and 56.

In still a further preferred embodiment, especially where doses are administered on consecutive days after the safety period, 30 to 40 mg/kg and especially 34 to 38 mg/kg are administered. Preferably such doses are administered as a first dosage as well as five additional doses on consecutive days following the safety period.

The pharmaceutical composition is preferably injected. This injection is administered using intravenous infusions, subcutaneously or intramuscular.

Further the pharmaceutical composition may comprise other pharmaceutically acceptable carriers and/or excipients. The term "pharmaceutically acceptable" means generally recognized pharmacopoeia for use in animals, and more particularly in humans.

The term "carrier" refers to a diluent, adjuvant, or vehicle with which the pharmaceutical composition is administered. Such pharmaceutical carriers can be sterile liquids, such as water and oils. Water is a preferred carrier when the pharmaceutical composition is administered intravenously. Saline solutions and aqueous dextrose and glycerol solutions can also be employed as liquid carriers, particularly for injectable solutions.

Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

Furthermore, the pharmaceutical composition may be administered in combination with one or more other therapeutic agent(s) or antibody or antibodies, such as steroids or intravenous immunoglobulin, in particular corticosteroids, glucocorticoid prodrugs, e.g. prednisone, IVIG, anti-D, vinca alkaloids, e.g. vincristine or vinblastine, danazol, immunosuppressive agents, e.g. azathioprine, cyclophosphamide or cyclosporin A, dapsone, thrombopoicetic agents, rituximab, mycophenolate mofetil, romiplostim, eltrombopag, mycophenolate mofetil.

As used herein, the term "in combination" refers to the use of more than one prophylactic and/or therapeutic agent. The use of the term "in combination" does not restrict the order in which prophylactic and/or therapeutic agents are administered to a patient.

The present invention provides kits that can be used including the pharmaceutical composition. It is also well known by a person skilled in the art that the pharmaceutical composition can be in the form of a multiple-dosage-kit containing sufficient amounts of administration doses of soluble FcγR for effectively treating or preventing inflammatory diseases and/or autoimmune diseases in a patient. In one embodiment, the pharmaceutical pack or kit comprises one or more containers filled with the pharmaceutical composition of the invention. Furthermore, one or more additional prophylactic or therapeutic agents useful for the treatment of a disease can also be included in the pharmaceutical pack or kit.

### BRIEF DESCRIPTION OF THE FIGURE AND THE SEQUENCES

Figure 1 shows the platelet response in Phase Ib with 12mg/kg/week SM101 versus placebo.

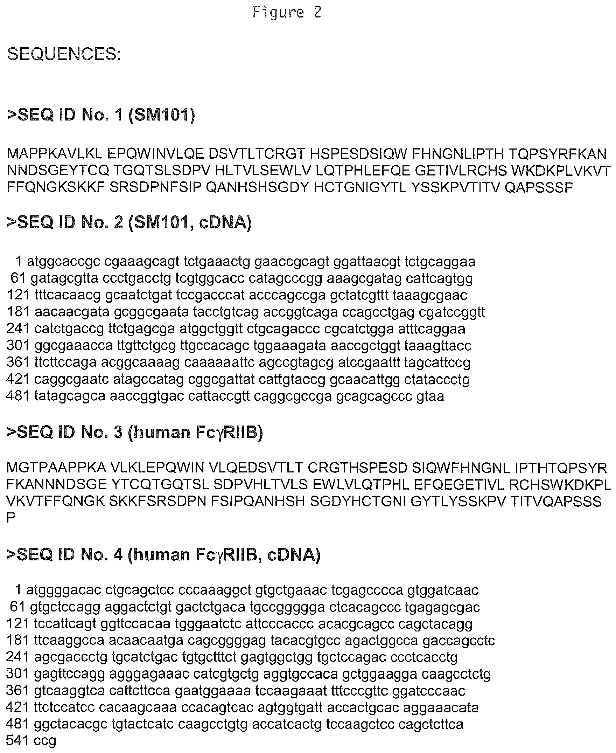

Figure 2 shows the following sequences:
   SEQ ID NO.1 shows the amino acid sequence of SM101.
   SEQ ID NO.2 shows the nucleic acid sequence coding SM101.
   SEQ ID NO.3 shows the amino acid sequence of human FcγRIIB.
   SEQ ID NO.4 shows the nucleic acid sequence coding human FcγRIIB.

### EXAMPLE

### Background

Primary Immune Thromocytopenia (ITP) is an autoimmune disorder characterized by a low platelet count below 100 x 10⁹/L and an exclusion of secondary causes such as bacterial infections or hematological malignancies. Fcγ-receptors are involved in the clearance of immune complexes, the antibody-dependent cellular cytotoxicity, the secretion of mediators and indirectly the regulation of B cell proliferation and differentiation. SM101 has been investigated successfully in autoimmune models, toxicology studies in patients with ITP and Systemic Lupus Erythematosus (SLE).

### Objectives

The primary objective was to evaluate the safety and tolerability of SM101 at increasing dose levels in patients with chronic ITP. Secondary objectives included efficacy in terms of platelet count and pharmacokinetic (PK) evaluation of SM101.

### Methods

The Ib part of the clinical trial was designed as a randomized, double-blind, placebo-controlled, multi-center dose escalation study in 36 ITP patients. Each dosing cohort consisted of 4 verum and 2 placebo subjects. Major inclusion criteria included a diagnosis of chronic ITP for at least 6 months and a platelet count of less than 30 x 10⁹/L at baseline. Concomitant corticosteroids were allowed at constant dose during the study. Eligible patients were dosed on day 1 with a 5 week safety period, followed up by the intended treatment cycle of 4 weekly infusions between day 35 and 56 and a final follow-up of 3 months.

### Results

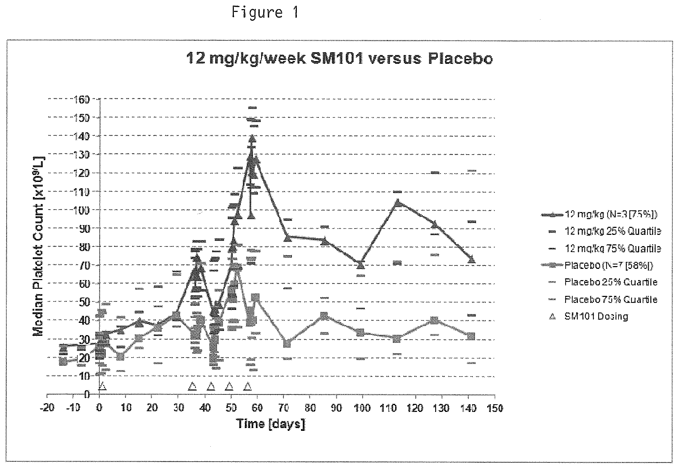

In total, 36 ITP patients in 6 dose groups received 5 infusions of 0.3 to 12,0 mg/kg/week SM101 or placebo. 56% of the patients were females, 39% of the patients were splenectomized and 39% received concomitant corticosteroids prior to randomization. In the placebo group. 42% received rescue medication compared to 0 to 25% in the higher dose groups with SM101. SM101 plasma levels increased proportionally with dose and the mean t1/2 across all dose groups was 42.7 h with a range of 14.0 to 99.9 h. In terms of immunogenicity, no anti-drug-antibodies against SM101 were observed. For patients without ITP rescue therapy, the highest dose group with 12 mg/kg/week SM101 showed a sustained median platelet response over 50 x 10⁹/L in comparison to placebo after one treatment cycle with 4 infusions (Figure 1). This response persisted during the 3 months follow-up period with a median platelet count of approximately 70 x 10⁹/L at the end of the clinical trial. The platelet response in the 12/mg/kg/week group started approximately 20 days after the beginning of the treatment cycle.

### Conclusions

To the inventors knowledge, this is the first time that a soluble Fcγ receptor showed efficacy in a clinical trial. In the 12 mg/kg/week group, SM101 showed a clear platelet response in patients with ITP.

The duration of the platelet response persisted for at least 3 months after the last administration of SM101. Multiple doses of SM101 up to 12 mg/kg/week were well tolerated with no DLT or SAE.
i Rodeghiero F, S. R. et al, (2009) Standardization of terminology, definitions and outcome criteria in immune thrombocytopenic purpura of adults and children: report of an international working group. Blood, 113 (11), 2386-2393.
ii Cines D.B. and Bussel J.B, (2005) How I treat: ITP. Blood, 106 (7), 2244-2251
iii SuppreMol, Public Summary of Positive Opinion for Orphan Designation of recombinant human soluble FC-gamma receptor IIb, EMEA/COMP/143892/2008, 29^{th} July 2008
iv Segal, J.B. & Powe, N.R., (2006) Prevalence of immune thrombocytopenia: analyses of administrative data. Journal of Thrombosis and Haemostasis, 4, 2377-2383.
v Feudjo-Tepie, M.A. et al, (2008) Prevalence of diagnosed chronic immune thrombocytopenic purpura in the US: analysis of a large US claim database: a rebuttal. Journal of Thrombosis and Haemostasis, 6, 711-712.
vi George, J.N. et al, (1996) Idiopathic Thrombocytopenic Pupura: A Practice Guideline Developed by Explicit Methods for the American Society of Hematology.
vii Brighton T.A. et al, (1996) Prospective evaluation of the clinical usefulness of an antigen-specific assay (MAIPA) in the idiopathic thrombocytopenic purpura and other immune thrombocytopenias. Blood, 88 (1), 194-201
viii British Society for Haematology; Guidelines for the Investigation and Management of Idiopathic thrombocytopenic purpura in adults, children and in pregnancy. British Journal of Haematology, 2003, 120, 574-596
ix Baccarani, U. et al, (1999) Splenectomy in hematology. Current practice and new perspectives. Haematologica, 84, 431-436.
x Godeau, B. et al, (2003) Treatment of adult chronic autoimmune thrombocytopenic purpura with repeated high-dose intravenous immunoglobulin. Blood, 82, 1415-1421.
xi Scaradavou, A. et al, (1997) Intravenous anti-D treatment of immune thrombocytopenic purpura: experience in 272 patients. Blood, 89, 2689-2700.
xii Berchtold, P. & McMillan, R. (1989) Therapy of chronic idiopathic thrombocytopenic purpura in adults. Blood, 74, 2309-2317.
xiii Ahn, Y.S. et al, (1989) Long-term danazol therapy in autoimmune thrombocytopenia: unmaintained remission and age-dependent response in women. Annals of Internal Medicine, 111, 723-729.
xiv Godeau, B.et al, (1997) Dapsone for chronic autoimmune thrombocytopenic purpura: a report of 66 cases. British Journal of Haematology, 97, 336-339
xv Burzynski J., (2009) New options after first-line therapy for chronic immune thrombocytopenic purpura. American Journal of Health-System Pharmacy, 66, S11-S21
xvi Provan D. et al, (2006) Efficacy of mycophenolate mofetil as single-agent therapy for refractory immune thrombocytopenic purpura. American Journal of Hematology, 81 (1), 19-25

## Claims

1. Pharmaceutical composition for use in treating or preventing either one or both of an inflammatory disease and an autoimmune disease, wherein
the pharmaceutical composition comprises soluble Fcγ receptor (FcγR) as active ingredient; and wherein the pharmaceutical composition is designed for administering a first dose of a therapeutically effective amount of soluble FcγR, followed by a safety period of several weeks, followed by a subsequent treatment cycle of at least two further administrations, wherein said therapeutically effective amount is effective to treat or prevent said disease in said patient.

2. Pharmaceutical composition of claim 1 , wherein the disease is Primary Immune Thrombocytopenia (ITP), Systemic Lupus Erythematosus (SLE), Rheumatoid Arthritis (RA) or Autoimmune Haemolytic Anaemia (AIHA).

3. Pharmaceutical composition of any one of the preceding claims, wherein the soluble FcγR is derived from FcγRIIb type receptor.

4. Pharmaceutical composition of any one of the preceding claims, wherein the soluble FcγR is SM101.

5. Pharmaceutical composition of any one of the preceding claims, wherein the therapeutically effective amount per administration is from 1 to 100 mg/kg, or is at least 3 mg/kg, or is from 3 to 50 mg/kg, or is from 5 to 30 mg/kg, or
is from 8 to 25 mg/kg, or
is from 12 to 20 mg/kg.

6. Pharmaceutical composition of any one of the preceding claims, wherein said administering is performed on day 1, followed by a 2 to 8 week safety period, followed by a subsequent treatment cycle of 2 to 7 weekly administrations between days 30 and 60, or
is performed on day 1, followed by a 4 to 6 week safety period, followed by a subsequent treatment cycle of 3 to 5 weekly administrations between days 35 and 60, or
is performed on day 1 , followed by a 5 week safety period, followed by a subsequent treatment cycle of 4 weekly administrations between days 35 and 56, or
is performed on day 1, followed by a 2 to 8 week safety period, followed by a subsequent treatment cycle of 2 to 7 administrations on consecutive days.

7. Pharmaceutical composition of any one of the preceding claims, wherein said soluble FcγR is of human origin.

8. Pharmaceutical composition of any one of the preceding claims, wherein said soluble FcγR is recombinant, non-glycosylated soluble FcγR.

9. Pharmaceutical composition of any one of the preceding claims, wherein said soluble FcγR is **characterized by** the absence of transmembrane domains and signal peptide.

10. Pharmaceutical composition of any one of the preceding claims, wherein said soluble FcγR contains the amino acids according to SEQ ID NO:1 or SEQ ID NO:3, or wherein said soluble FcγR contains a nucleic acid sequence as shown in SEQ ID NO:2 or SEQ ID NO: 4.

11. Pharmaceutical composition of any one of the preceding claims, wherein administration of the pharmaceutical composition is performed by injections or by infusions.

12. Pharmaceutical composition of any one of the preceding claims, wherein the administration of the pharmaceutical composition is performed intravenously, subcutaneously or intramuscularly.

13. Pharmaceutical composition of any one of the preceding claims, wherein the pharmaceutical composition comprises pharmaceutically acceptable carriers and/or excipients.

14. Pharmaceutical composition of any one of the preceding claims, wherein the pharmaceutical composition is administered in combination with one or more other pharmaceutical agents and/or antibodies.

15. Pharmaceutical composition of any one of the preceding claims, wherein said pharmaceutical composition is in the form of a multiple-dosage-kit containing sufficient amounts of administration doses of soluble FcγR for effectively treating or preventing inflammatory diseases and/or autoimmune diseases in a patient.

## Patentansprüche

1. Pharmazeutische Zusammensetzung zur Verwendung beim Behandeln oder Verhindern einer entzündlichen Erkrankung und/oder einer Autoimmunkrankheit, wobei
die pharmazeutische Zusammensetzung einen löslichen Fcγ-Rezeptor (FcγR) als Wirkstoff umfasst; und wobei die pharmazeutische Zusammensetzung dafür ausgelegt ist, eine erste Dosis einer therapeutisch wirksamen Menge an löslichem FcγR zu verabreichen, gefolgt von einem mehrwöchigen Sicherheitszeitraum, gefolgt von einem darauf folgenden Behandlungszyklus von zumindest zwei weiteren Verabreichungen, wobei die therapeutisch wirksame Menge wirksam ist, um die Krankheit bei dem Patienten zu behandeln oder zu verhindern.

2. Pharmazeutische Zusammensetzung nach Anspruch 1, wobei es sich bei der Krankheit um primäre Immunthrombozytopenie (ITP), systemischen Lupus erythematodes (SLE), rheumatoide Arthritis (RA) oder autoimmune hämolytische Anämie (AIHA) handelt.

3. Pharmazeutische Zusammensetzung nach einem der vorhergehenden Ansprüche, wobei der lösliche FcγR von einem Rezeptor vom FcγRIIb-Typ abgeleitet ist.

4. Pharmazeutische Zusammensetzung nach einem der vorhergehenden Ansprüche, wobei es sich bei dem löslichen FcγR um SM101 handelt.

5. Pharmazeutische Zusammensetzung nach einem der vorhergehenden Ansprüche, wobei die therapeutisch wirksame Menge pro Verabreichung von 1 bis 100 mg/kg beträgt oder zumindest 3 mg/kg beträgt oder von 3 bis 50 mg/kg beträgt oder von 5 bis 30 mg/kg beträgt oder
von 8 bis 25 mg/kg beträgt oder
von 12 bis 20 mg/kg beträgt.

6. Pharmazeutische Zusammensetzung nach einem der vorhergehenden Ansprüche, wobei das Verabreichen an Tag 1 durchgeführt wird, gefolgt von einem 2- bis 8-wöchigen Sicherheitszeitraum, gefolgt von einem darauf folgenden Behandlungszyklus aus 2 bis 7 wöchentlichen Verabreichungen zwischen Tag 30 und 60 oder
an Tag 1 durchgeführt wird, gefolgt von einem 4- bis 6-wöchigen Sicherheitszeitraum, gefolgt von einem darauf folgenden Behandlungszyklus aus 3 bis 5 wöchentlichen Verabreichungen zwischen Tag 35 und 60 oder
an Tag 1 durchgeführt wird, gefolgt von einem 5-wöchigen Sicherheitszeitraum, gefolgt von einem darauf folgenden Behandlungszyklus aus 4 wöchentlichen Verabreichungen zwischen Tag 35 und 56 oder
an Tag 1 durchgeführt wird, gefolgt von einem 2- bis 8-wöchigen Sicherheitszeitraum, gefolgt von einem darauf folgenden Behandlungszyklus aus 2 bis 7 Verabreichungen an aufeinanderfolgenden Tagen.

7. Pharmazeutische Zusammensetzung nach einem der vorhergehenden Ansprüche, wobei der lösliche FcγR menschlichen Ursprungs ist.

8. Pharmazeutische Zusammensetzung nach einem der vorhergehenden Ansprüche, wobei es sich bei dem löslichen FcγR um einen rekombinanten, nicht-glykosylierten löslichen FcγR handelt.

9. Pharmazeutische Zusammensetzung nach einem der vorhergehenden Ansprüche, wobei der lösliche FcγR durch das Fehlen von Transmembrandomänen und eines Signalpeptids gekennzeichnet ist.

10. Pharmazeutische Zusammensetzung nach einem der vorhergehenden Ansprüche, wobei der lösliche FcγR die Aminosäuren nach SEQ ID NR. 1 oder SEQ ID NR. 3 enthält oder wobei der lösliche FcγR eine Nukleinsäuresequenz enthält, wie sie in SEQ ID NR. 2 oder SEQ ID NR. 4 gezeigt ist.

11. Pharmazeutische Zusammensetzung nach einem der vorhergehenden Ansprüche, wobei die Verabreichung der pharmazeutischen Zusammensetzung durch Injektionen oder Infusionen durchgeführt wird.

12. Pharmazeutische Zusammensetzung nach einem der vorhergehenden Ansprüche, wobei die Verabreichung der pharmazeutischen Zusammensetzung intravenös, subkutan oder intramuskulär durchgeführt wird.

13. Pharmazeutische Zusammensetzung nach einem der vorhergehenden Ansprüche, wobei die pharmazeutische Zusammensetzung pharmazeutisch akzeptable Träger und/oder Hilfsstoffe umfasst.

14. Pharmazeutische Zusammensetzung nach einem der vorhergehenden Ansprüche, wobei die pharmazeutische Zusammensetzung in Kombination mit einem oder mehreren anderen pharmazeutischen Wirkstoffen und/oder Antikörpern verabreicht wird.

15. Pharmazeutische Zusammensetzung nach einem der vorhergehenden Ansprüche, wobei die pharmazeutische Zusammensetzung in der Form eines Mehrfachdosierungskits vorliegt, das ausreichende Mengen an Verabreichungsdosen von löslichem FcγR enthält, um entzündliche Erkrankungen und/oder Autoimmunkrankheiten bei einem Patienten wirksam zu behandeln oder zu verhindern.

## Revendications

1. Composition pharmaceutique destinée à être utilisée pour le traitement ou la prévention d'une maladie inflammatoire et/ou d'une maladie auto-immune, dans laquelle la composition pharmaceutique comprend un récepteur Fcγ (FcγR) soluble en tant que principe actif, et dans laquelle la composition pharmaceutique est destinée à l'administration d'une première dose d'une quantité thérapeutiquement efficace de FcγR soluble, suivie par une période d'innocuité de plusieurs semaines, suivie par un cycle de traitement subséquent d'au moins deux autres administrations supplémentaires, ladite quantité thérapeutiquement efficace étant efficace pour traiter ou prévenir ladite maladie chez ledit patient.

2. Composition pharmaceutique selon la revendication 1, dans laquelle la maladie est la thrombocytopénie immune primaire (ITP), le lupus érythémateux systémique (SLE ), la polyarthrite rhumatoïde (RA) ou l'anémie hémolytique auto-immune (AIHA)

3. Composition pharmaceutique selon l'une quelconque des revendications précédentes, dans laquelle le FcγR soluble est dérivé du récepteur de type FcγRIIb

4. Composition pharmaceutique selon l'une quelconque des revendications précédentes, dans laquelle le FcγR soluble est SM101.

5. Composition pharmaceutique selon l'une quelconque des revendications précédentes, dans laquelle la quantité thérapeutiquement efficace par administration est de 1 à 100 mg/kg, ou est au moins de 3 mg/kg, ou est de 3 à 50 mg/kg, ou est de 5 à 30 mg/kg, ou
est de 8 à 25 mg/kg, ou
est de 12 à 20 mg/kg.

6. Composition pharmaceutique selon l'une quelconque des revendications précédentes, dans laquelle ladite administration est réalisée le jour 1, suivie par une période d'innocuité de 2 à 8 semaines, suivie par un cycle de traitement subséquent de 2 à 7 administrations par semaine entre les jours 30 et 60, ou
est réalisée le jour 1, suivie par une période d'innocuité de 4 à 6 semaines, suivie par un cycle de traitement subséquent de 3 à 5 administrations par semaine entre les jours 35 et 60, ou
est réalisée le jour 1, suivie par une période d'innocuité de 5 semaines, suivie par un cycle de traitement subséquent de 4 administrations par semaine entre les jours 35 et 56, ou
est réalisée le jour 1, suivie par une période d'innocuité de 2 à 8 semaines, suivie par un cycle de traitement subséquent de 2 à 7 administrations sur des jours consécutifs.

7. Composition pharmaceutique selon l'une quelconque des revendications précédentes, dans laquelle ledit FcγR soluble est d'origine humaine.

8. Composition pharmaceutique selon l'une quelconque des revendications précédentes, dans laquelle ledit FcγR soluble est du FcγR soluble non-glycosylé recombinant.

9. Composition pharmaceutique selon l'une quelconque des revendications précédentes, dans laquelle ledit FcγR soluble est **caractérisé par** l'absence de domaines transmembranaires et de peptide signal.

10. Composition pharmaceutique selon l'une quelconque des revendications précédentes, dans laquelle ledit FcγR soluble contient les acides aminés selon la SEQ ID N°1 ou la SEQ ID N°3, ou dans laquelle ledit FcγR soluble contient une séquence d'acides nucléiques telle que représentée dans la SEQ ID N°2 ou la SEQ ID N°4.

11. Composition pharmaceutique selon l'une quelconque des revendications précédentes, dans laquelle l'administration de la composition pharmaceutique est réalisée par injections ou par infusions.

12. Composition pharmaceutique selon l'une quelconque des revendications précédentes, dans laquelle l'administration de la composition pharmaceutique est réalisée par une méthode intraveineuse, sous-cutanée ou intramusculaire.

13. Composition pharmaceutique selon l'une quelconque des revendications précédentes, dans laquelle la composition pharmaceutique comprend des supports et / ou excipients pharmaceutiquement acceptables.

14. Composition pharmaceutique selon l'une quelconque des revendications précédentes, dans laquelle la composition pharmaceutique est administrée en combinaison avec un ou plusieurs autres agents pharmaceutiques et/ou anticorps.

15. Composition pharmaceutique selon l'une quelconque des revendications précédentes, dans laquelle ladite composition pharmaceutique est sous forme d'une trousse à doses multiples, qui comprend des quantités suffisantes de doses d'administration de FcγR soluble pour traiter ou prévenir efficacement des maladies inflammatoires et/ou des maladies auto-immunes chez un patient.
